# EUROPÄISCHE PATENTSCHRIFT

(11) **EP 0 804 142 B1**
(45) Veröffentlichungstag und Bekanntmachung des Hinweises auf die Patenterteilung: **17.03.1999**
(21) Anmeldenummer: 96900922.4
(22) Anmeldetag: 09.01.1996
(51) Int. Cl.: A61K 7/13, D06P 3/04

(54) **DIKETO-VERBINDUNGEN ZUM FÄRBEN KERATINHALTIGER FASERN**
DIKETO COMPOUNDS FOR COLOURING KERATIN-CONTAINING FIBRES
COMPOSES DICETO POUR COLORER DES FIBRES CONTENANT DE LA KERATINE

(30) Priorität: 18.01.1995 DE 19501302
(43) Veröffentlichungstag der Anmeldung: 05.11.1997
(73) Patentinhaber: Henkel Kommanditgesellschaft auf Aktien, 40191 Düsseldorf (DE)
(72) Erfinder: MÖLLER, Hinrich, D-40789 Monheim (DE); HÖFFKES, Horst, D-40595 Düsseldorf (DE)
(86) Internationale Anmeldenummer: EP9600057
(87) Internationale Veröffentlichungsnummer: WO9622076

(56) Entgegenhaltungen:
- EP-A- 0 020 274
- DE-A- 4 335 627

## Beschreibung

Gegenstand der Erfindung ist die Verwendung Von bestimmten Diketo-Verbindungen zum Färben von keratinhaltigen Fasern, insbesondere menschlichen Haaren.

Für das Färben von keratinhaltigen Fasern, z. B. Haaren, Wolle oder Pelzen, kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung. Mit Oxidationsfarbstoffen lassen sich zwar intensive Färbungen mit guten Echtheitseigenschaften erzielen, die Entwicklung der Farbe geschieht jedoch unter dem Einfluß von Oxidationsmitteln wie z. B. H₂O₂, was häufig Schädigungen der Faser zur Folge hat. Direktziehende Farbstoffe werden unter schonenderen Bedingungen appliziert, ihr Nachteil liegt jedoch darin, daß die Färbungen häufig nur über unzureichende Echtheitseigenschaften verfügen.

Überraschenderweise wurde nun gefunden, daß sich bestimmte Diketo-Verbindungen auch in Abwesenheit von oxidierenden Agentien hervorragend zum Färben von keratinhaltigen Fasern eignen. Der Einsatz von oxidierenden Agentien soll dabei jedoch keineswegs prinzipiell ausgeschlossen werden.

Gegenstand der EP-A-0020274 sind Haarfarbemittel mit Curcumaextrakten, die als färbenden Stoff Curcumin enthalten.

Die Verwendung der unten näher beschriebenen Diketo-Verbindungen zum Färben von keratinhaltigen Fasern ist bislang nicht bekannt.

Als keratinhaltige Fasern kommen z.B. Wolle, Pelze, Felle und menschliche Haare in Betracht. Die unten näher bezeichneten Diketo-Verbindungen können prinzipiell aber auch zum Färben anderer Naturfasern, wie z.B. Baumwolle, Jute, Sisal, Leinen oder Seide, modifizierter Naturfasern, wie z.B. Regeneratcellulose, Nitro-, Alkyl- oder Hydroxyalkyl- oder Acetylcellulose und synthetischer Fasern, wie z.B. Polyamid-, Polyacrylnitril,-, Polyurethan- und Polyesterfasern verwendet werden.

Gegenstand der Erfindung ist die Verwendung von Diketo-Verbindungen der Formel I 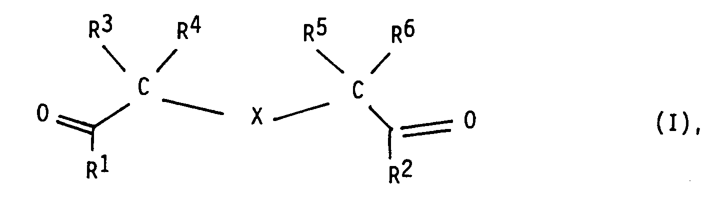 in der die Reste R¹ und R² C₁-C₄-Alkylgruppen oder Arylgruppen bedeuten, die Reste R³ bis R⁶ für Wasserstoffe oder C₁-C₄-Alkylgruppen stehen, oder aber einer der Reste R³ und R⁴ mit einem der Reste R⁵ und R⁶ verbunden ist, so daß ein isocyclischer oder heterocyclischer, gesättigter oder ungesättigter 5-, 6- oder 7-Ring resultiert, und X für eine Methylen-, Ethylen- oder Vinylengruppe, eine N(H)-Gruppe oder ein S- oder O-Atom steht, sowie deren Ketale zum Färben von keratinhaltigen Fasern, insbesondere menschlichen Haaren.

Als Beispiele für die erfindungsgemäß einzusetzenden Diketo-Verbindungen können genannt werden: 2,6-Heptandion, 1,3-Dibenzoylpropan, 2,5-Diacetylfuran, 2,5-Diacetylthiophen, 2,6-Diacetylpyridin.

Bevorzugte Verwendung finden 2,6-Diacetylpyridin und 1,3-Dibenzoylpropan.

Diese Substanzen sind i.a. literaturbekannt oder im Chemikalienhandel erhältlich.

Die Diketo-Verbindungen der Formel I alleine färben Keratinfasern nur schwach gelb. Brillante Färbungen im Orange-, Braun-, Rotbraun-, Blauschwarz- und Schwarzbereich mit guten Echtheitseigenschaften (Lichtechtheit, Waschechtheit, Reibechtheit) werden erzielt, wenn die Diketo-Verbindungen der Formel I gemeinsam mit Verbindungen mit primärer oder sekundärer Aminogruppe, z.B. Anilinderivaten, mit einer Stickstoff enthaltenden heterocyclischen Verbindung, z.B. primären heteroaromatischen Aminen, oder einer aromatischen Hydroxyverbindung verwendet werden. Dies sind einerseits Verbindungen, die für sich alleine Haare ebenfalls nur schwach färben und erst gemeinsam mit den Diketo-Verbindungen der Formel I brillante Färbungen ergeben. Andererseits sind darunter aber auch Verbindungen, die als Oxidationsfarbstoffvorprodukte Verwendung finden und für sich allein zum Haarefärben geeignet sind; in Gegenwart der Diketo-Verbindungen der Formel I jedoch werden noch kräftigere, brillantere Nuancen mit noch besseren Echtheitseigenschaften erzielt.

In den Rahmen der vorliegenden Erfindung fällt auch die Verwendung solcher Substanzen, die Reaktionsprodukte von Diketo-Verbindungen der Formel I mit den genannten Verbindungen darstellen.

Geeignete Verbindungen mit primärer oder sekundärer Aminogruppe sind z.B. primäre aromatische Amine wie N-(2-Hydroxyethyl)-N-ethyl-, N,N-Bis(2-hydroxyethyl)-, N-(2-Methoxyethyl-), 2,3-, 2,4-, 2,5-Dichlor-p-phenylendiamin, 2-Chlor-p-phenylendiamin, 2,5-Dihydroxy-4-morpholinoanilin-dihydrobromid, 2-, 3-, 4-Aminophenol, o-, m-, p-Phenylendiamin, 2,5-Diaminotoluol, -phenol, -anisol, -phenethol, 2-Chlor-p-phenylendiamin, 4-Methylamino-, 3-, 4-Dimethylamino-, 3,4-Methylendioxyanilin, 3-Amino-2,4-dichlor-, 4-Methylamino-, 2-Methyl-5-amino-, 3-Methyl-4-amino-, 2-Methyl-5-(2-hydroxyethylamino)-, 2-Methyl-5-amino-6-chlor-, 2-Methyl-5-amino-4-chlor-, 2-Methyl-5-amino-6-chlor-, 5-(2-Hydroxyethylamino)-4-methoxy-2-methyl-, 4-Amino-2-aminomethyl-phenol, 1,3-Diamino-2,4-dimethoxybenzol, 2-, 3-, 4-Aminobenzoesäure, -phenylessigsäure, 2,3-, 2,4-, 2,5-, 3,4-, 3,5-Diaminobenzoesäure, 4-, 5-Aminosalicylsäure, 3-Amino-4-hydroxy-, 4-Amino-3-hydroxy-benzoesäure, 2-, 3-, 4-Aminobenzolsulfonsäure, 3-Amino-4-hydroxybenzolsulfonsäure, 4-Amino-3-hydroxynaphthalin-1-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 1,3,5-, 1,2,4-Triaminobenzol, 1,2,4,5-Tetraaminobenzol-tetrahydrochlorid, 2,4,5-Triaminophenol-trihydrochlorid, Pentaaminobenzol-pentahydrochlorid, Hexamaminobenzol-hexahydrochlorid, 2,4,6-Triaminoresorcin-trihydrochlorid, 4,5-Diaminobrenzcatechinsulfat, 4,6-Diaminopyrogallol-dihydrochlorid, 3,5-Diamino-4-hydroxybrenzcatechin-sulfat, aromatische Aniline bzw. Phenole mit einem weiteren aromatischen Rest wie 4,4'-Diaminostilben-dihydrochlorid, 4,4'-Diaminostilben-2,2'-disulfonsäure, Na-Salz, 4,4'-Diaminodiphenylmethan, -sulfid, -sulfoxid, -amin, 4,4'-Diaminodiphenylamin-2-sulfonsäure, 4,4'-Diaminobenzophenon, -diphenylether, 3,3',4,4'-Tetraaminodiphenyl-tetrahydrochlorid, 3,3',4,4'-Tetraamino-benzophenon, 1,3-Bis-(2,4-diaminophenoxy)-propan-tetrahydrochlorid, 1,8-Bis-(2,5-diaminophenoxy)-3,6-dioxaoctan-tetrahydrochlorid, 1,3-Bis-(4-aminophenylamino)-propan, -2-propanol, 1,3-Bis-[N-(4-aminophenyl)-2-hydroxyethylamino]-2-propanol, N,N-Bis-[2-(4-aminophenoxy)-ethyl]-methylamin-trihydrochlorid.

Geeignete stickstoffhaltige Heterocyclen sind z.B. 2-, 3-, 4-Amino-, 2-Amino-3-hydroxy-, 2,6-Diamino-, 2,5-Diamino-, 2,3-Diamino-, 2-Dimethylamino-5-amino-, 3-Amino-2-methylamino-6-methoxy-, 2,3-Diamino-6-methoxy-, 3,5-Diamino-2,6-dimethoxy-, 2,4,5-Triamino-, 2,6-Dihydroxy-3,4-dimethylpyridin, 4,5,6-Triamino-, 4-Hydroxy-2,5,6-triamino-, 2,4,5,6-Tetraamino-, 2-Methylamino-4,5,6-triamino, 2,4-, 4,5-Diamino-, 2-Amino-4-methoxy-6-methyl-pyrimidin, 3,5-Diaminopyrazol, -1,2,4-triazol, 3-Amino-, 3-Amino-5-hydroxypyrazol, 2-,3-, 8-Aminochinolin, 4-Amino-chinaldin, 2-, 6-Aminonicotinsäure, 5-Aminoisochinolin, 4-, 5-, 6-, 7-Aminoindol, 5-, 6-Aminoindazol, 5-, 7-Amino-benzimidazol, -benzothiazol, 2,5-Dihydroxy-4-morpholinoanilin sowie Indol- und Indolinderivate, z.B. 5,6-Dihydroxyindol, 5,6-Dihydroxyindolin und 4-Hydroxyindolin, sowie deren mit vorzugsweise anorganischen Säuren wie z. B. Salz- oder Schwefelsäure gebildeten Salze.

Geeignete aromatische Hydroxyverbindungen sind z.B. 2-, 4-, 5-Methylresorcin, Resorcin, 3-Methoxyphenol, Brenzkatechin, Hydrochinon, Pyrogallol, Phloroglucin, Hydroxyhydrochinon, 2-, 3-, 4-Methoxy-, 3-Dimethylamino-, 2-(2-Hydroxyethyl)-, 3,4-Methylendioxyphenol, 2,4-, 3,4-Dihydroxybenzoesäure, -phenylessigsäure, Gallussäure, 2,4,6-Trihydroxybenzoesäure, -acetophenon, 2-, 4-Methyl-, 2-, 4-Chlorresorcin, 1-, 2-Naphthol, 1,5-, 2,3-, 2,7-Dihydroxynaphthalin, 6-Dimethylamino-4-hydroxy-2-naphthalinsulfonsäure, 3,6-Dihydroxy-2,7-naphthalinsulfonsäure.

Auch Aminosäuren oder aus 2 bis 9 Aminosäuren aufgebaute Oligopeptide sind geeignet. Als Aminosäuren kommen alle natürlich vorkommenden und synthetischen Aminosäuren in Frage, z.B. die durch Hydrolyse aus pflanzlichen oder tierischen Proteinen, z.B. Kollagen, Keratin, Casein, Elastin, Sojaprotein, Weizengluten oder Mandelprotein zugänglichen Aminosäuren. Dabei können sowohl sauer als auch alkalisch reagierende Aminosäuren eingesetzt werden. Geeignete Oligopeptide sind alle aus natürlich vorkommenden und synthetischen Aminosäuren aufgebauten Oligopeptide.

Die Oligopeptide können dabei natürlich vorkommende oder synthetische Oligopeptide, aber auch die in Polypeptid- oder Proteinhydrolysaten enthaltenen Oligopeptide sein, sofern sie über eine für die Anwendung in den erfindungsgemäßen Färbemitteln ausreichende Wasserlöslichkeit verfügen. Als Beispiele sind z.B. Glutathion oder die in den Hydrolysaten von Kollagen, Keratin, Casein, Elastin, Sojaprotein, Weizengluten oder Mandelprotein enthaltenen Oligopeptide zu nennen.

Bevorzugt ist dabei die Verwendung gemeinsam mit Verbindungen mit primärer oder sekundärer Aminogruppe oder mit aromatischen Hydroxyverbindungen.

In allen Färbemitteln können auch mehrere verschiedene Diketo-Verbindungen der Formel I gemeinsam zum Einsatz kommen; ebenso können auch mehrere verschiedene Komponenten aus den Gruppen von Verbindungen mit primärer oder sekundärer Aminogruppe, von stickstoffhaltigen Heterocyclen, aromatischen Hydroxyverbindungen oder Aminosäuren gemeinsam verwendet werden.

Die Färbemittel ergeben bereits bei physiologisch verträglichen Temperaturen von unter 45°C intensive Färbungen. Sie eignen sich deshalb besonders zum Färben von menschlichen Haaren. Zur Anwendung auf dem menschlichen Haar können die Färbemittel in einen wasserhaltigen kosmetischen Träger oder auch in einem wasserfreien Träger eingearbeitet werden. Geeignete wasserhaltige kosmetische Träger sind z.B. Cremes, Emulsionen, Gele oder auch tensidhaltige schäumende Lösungen wie z.B. Shampoos oder andere Zubereitungen, die für die Anwendung auf dem Haar geeignet sind.

Der wasserhaltige kosmetische Träger enthält üblicherweise Netz- und Emulgiermittel wie anionische, nichtionische oder ampholytische Tenside, z.B. Fettalkoholsulfate, Alkansulfonate, α-Olefinsulfonate, Fettalkoholpolyglykolethersulfate, Alkylglycoside, Ethylenoxidanlagerungsprodukte an Fettalkohole, an Fettsäuren, an Alkylphenole, an Sorbitanfettsäureester, an Fettsäurepartialglyceride und Fettsäurealkanolamide; Verdickungsmittel, z. B. Fettalkohole, Fettsäuren, Paraffinöle, Fettsäureester und andere Fettkomponenten in emulgierter Form; wasserlösliche polymere Verdickungsmittel wie natürliche Gummen, z. B. Gummi arabicum, Karaya-Gummi, Guar-Gummi, Johannisbrotkernmehl, Leinsamengummen und Pektin, biosynthetische Gummen, z.B. Xanthan-Gummi und Dextrane, synthetische Gummen, z. B. Agar-Agar und Algin, Stärke-Fraktionen und Derivate wie Amylose, Amylopektin und Dextrine, modifizierte Cellulosemoleküle, z. B. Methylcellulose, Hydroxyalkylcellulose und Carboxymethylcellulose, Tone wie z. B. Bentonit oder vollsynthetische Hydrokolloide, z.B. Polyvinylalkohol oder Polyvinylpyrrolidon, haarpflegende Zusätze, wie z.B. wasserlösliche kationische Polymere, anionische Polymere, nichtionische Polymere, amphotere oder zwitterionische Polymere, Pantothensäure, Vitamine, Pflanzenextrakte oder Cholesterin, pH-Stellmittel, Komplexbildner und Parfumöle sowie Reduktionsmittel zur Stabilisierung der Inhaltsstoffe, z. B. Ascorbinsäure, schließlich können auch Farbstoffe zum Einfärben der kosmetischen Zubereitungen enthalten sein.

Außerdem kann der Zusatz von Lösungsvermittlern wie Ethylen, 1,3-Propylen-, 1,2-Propylen, 1,2-Butylenglykol, Glycerin, Ethanol, tert. Butanol, 2-Propanol oder Phenethol in Mengen von 2 - 50 % nützlich sein.

Auch die Anwendung aus wasserfreien Trägern ist geeignet, wenn das zu färbende Substrat mit Wasser angefeuchtet ist. Als wasserfreie Träger eignen sich z.B. mit wasserfreien Emulgatoren kombinierte Öle wie Paraffinöle oder Silikonöle niedriger bis mittlerer Viskosität.

Die Diketo-Verbindungen der Formel I sowie die Verbindungen mit primärer oder sekundärer Aminogruppe, die Aminosäuren oder aus 2 bis 9 Aminosäuren aufgebaute Oligopeptide, die stickstoffhaltigen Heterocyclen bzw. die aromatischen Hydroxyverbindungen sind dabei in einer Menge von jeweils 0,3 bis 65, vorzugsweise 6 bis 20 mMol, jeweils bezogen auf 100 g des gesamten Färbemittels, enthalten.

Für das Färbeergebnis kann es vorteilhaft sein, den Färbemitteln Ammonium- oder Metallsalze zuzugeben. Geeignete Metallsalze sind z.B. die Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate des Kaliums, Natriums, Lithiums, Magnesiums, Calciums, Strontiums, Bariums, Mangans, Eisens, Kobalts, Kupfers, Zinks; bevorzugt sind Natriumacetat, Lithiumbromid, Calciumbromid, Calciumgluconat, Zinkchlorid, Zinksulfat, Magnesiumchlorid, Magnesiumsulfat, Ammoniumcarbonat, -chlorid und -acetat, die im gegebenen Falle in einer Menge von 0,3 bis 65, vorzugsweise 6 bis 20 mMol, bezogen auf 100 g des gesamten Färbemittels, enthalten sind.

Der pH-Wert der gebrauchsfertigen Färbezubereitungen liegt zwischen 2 und 11, vorzugsweise zwischen 5 und 9.

Zum Haarefärben werden die Färbemittel in Form des wasserhaltigen, - oder auch des wasserfreien - kosmetischen Trägers in einer Menge von 50 - 100 g auf das Haar aufgebracht, ca. 30 Minuten dort belassen und dann ausgespült oder mit einem handelsüblichen Haarshampoo ausgewaschen.

Die beiden reaktiven Komponenten können entweder gleichzeitig auf das Haar aufgebracht werden oder aber auch nacheinander, wobei es keine Rolle spielt, welche der beiden Komponenten zuerst aufgetragen wird; die Ammonium- oder Metallsalze können dabei der ersten oder zweiten Komponente zugesetzt werden. Zwischen dem Auftragen der ersten und der zweiten Komponente können bis zu 30 Minuten Zeitabstand liegen. Auch eine Vorbehandlung der Haare mit der Salzlösung ist möglich.
Es besteht auch die Möglichkeit, die beiden reaktiven Komponenten zunächst zusammenzumischen und dann das Reaktionsprodukt auf die Haare aufzubringen. Gegebenenfalls kann das Reaktionsprodukt auch isoliert und dann als Farbstoff verwendet werden.

Die beiden reaktiven Komponenten können zusammen und getrennt entweder wasserfrei oder bereits in der fertigen Formulierung gelagert werden. Bei der getrennten Lagerung werden die reaktiven Komponenten erst unmittelbar vor der Anwendung miteinander innig vermischt. Dabei ist bei der trockenen Lagerung eine definierte Menge warmen (50 - 80°C) Wassers hinzuzufügen und eine homogene Mischung herzustellen. Werden die Komponente zusammen gelagert, dann liegen sie entweder in Pulverform oder in einer nichtwäßrigen flüssigen Grundlage vor. Diese kann z.B. aus Paraffinöl perliquidum oder einem geeigneten Silikonöl vergleichbarer Viskosität, gegebenenfalls unter Zusatz von Emulgatoren, bestehen. Die Applikation aus der nichtwäßrigen flüssigen Grundlage kann direkt auf dem feuchten Haar erfolgen.

Die folgenden Beispiele sollen den Erfindungsgegenstand näher erläutern, ohne ihn jedoch hierauf zu beschränken.

### Beispiele

### Herstellung einer Färbelosung:

Es wurde eine Aufschlämmung von 10 mMol einer Diketo-Verbindung der Formel I und 10 mMol eines Reaktants, 10 mMol Natriumacetat und ein Tropfen einer 20 %igen Fettalkylethersulfat-Lösung in 100 ml Wasser bereitet. Die Aufschlämmung wurde auf Siedetemperatur erhitzt und nach dem Abkühlen filtriert, der pH-Wert wurde anschließend auf 6 eingestellt.
In diese Färbelösung wurden bei 30°C 30 Minuten lang zu 90 % ergraute, nicht vorbehandelte Menschenhaare eingebracht. Die jeweiligen Färbetemperaturen, Färbedauern, Farbnuancen und Farbtiefen sind der Tabelle 1 zu entnehmen.

Die Farbtiefe wurde dabei nach folgender Skala bewertet:

| | |
|---|---|
| - | keine oder eine sehr blasse Ausfärbung |
| (+) | schwache Intensität |
| + | mittlere Intensität |
| +(+) | mittlere bis starke Intensität |
| ++ | starke Intensität |
| ++(+) | starke bis sehr starke Intensität |
| +++ | sehr starke Intensität |

**Tabelle 1**

| **Ausfärbungen mit 2,6-Diacetylpyridin** | | |
|---|---|---|
| Reaktant | Färbenuance | Farbtiefe |
| 2,5-Diaminotoluol · H₂SO₄ | mittelbraun | ++ |
| 2,4,5,6-Tetraaminopyrimidin · H₂SO₄ | gelb | +(+) |
| 1,8-Bis-(2,5-diaminophenoxy)-3,6-dioxaoctan · 4 HCl | dunkelgrau | ++(+) |
| 2-Methylamino-3-amino-6-methyloxypyridin · 2 HCl | schwarzbraun | +++ |
| 2-(2,5-Diaminophenyl)-ethanol · H₂SO₄ | mittelbraun | ++ |
| 2-Aminomethyl-4-aminophenol · 2 HCl | gelbbraun | +(+) |
| N,N-Bis-(2-hydroxyethyl)-p-phenylendiamin H₂SO₄ | mittelbraun | ++ |
| 4,4'-Diaminodiphenylamin · H₂SO₄ | schwarzblau | +++ |
| 2,6-Dimethoxy-3,5-diaminopyridin · 2 HCl | schwarz | +++ |

**Tabelle 2**

| **Ausfärbungen mit 1,3-Dibenzoylpropan** | | |
|---|---|---|
| Reaktant | Färbenuance | Farbtiefe |
| 2,5-Diaminotoluol · H₂SO₄ | dunkelviolett | +++ |
| 2,4,5,6-Tetraaminopyrimidin · H₂SO₄ | gelborange | ++(+) |
| 1,8-Bis-(2,5-diaminophenoxy)-3,6-dioxaoctan · 4 HCl | blauschwarz | +++ |
| 2-Methylamino-3-amino-6-methyloxypyridin · 2 HCl | dunkelbraun | +++ |
| 2-(2,5-Diaminophenyl)-ethanol · H₂SO₄ | grauviolett | ++(+) |
| 2-Aminomethyl-4-aminophenol · 2 HCl | grauviolett | +(+) |
| N,N-Bis-(2-hydroxyethyl)-p-phenylendiamin · H₂SO₄ | violettbraun | ++(+) |
| 4,4'-Diaminodiphenylamin · H₂SO₄ | blauschwarz | +++ |
| 2,6-Dimethoxy-3,5-diaminopyridin · 2 HCl | schwarz | +++ |

## Patentansprüche

1. Verwendung von Diketo-Verbindungen der Formel I 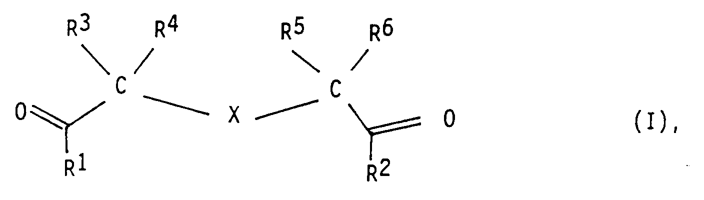 in der die Reste R¹ und R² C₁-C₄-Alkylgruppen oder Arylgruppen bedeuten, die Reste R³ bis R⁶ für Wasserstoffe oder C₁-C₄-Alkylgruppen stehen, oder aber einer der Reste R³ und R⁴ mit einem der Reste R⁵ und R⁶ verbunden ist, so daß ein isocyclischer oder heterocyclischer, gesättigter oder ungesättigter 5-, 6- oder 7-Ring resultiert, und X für eine Methylen-, Ethylen- oder Vinylengruppe, eine N(H)-Gruppe oder ein S- oder O-Atom steht, sowie deren Ketale zum Färben von keratinhaltigen Fasern, insbesondere menschlichen Haaren.

2. Verwendung nach Anspruch 1, dadurch gekennzeichnet, daß die Diketo-Verbindung der Formel I ausgewählt ist aus 2,6-Diacetylpyridin und 1,3-Dibenzoylpropan.

3. Verwendung nach Anspruch 1 und 2, dadurch gekennzeichnet, daß Verbindungen mit primärer oder sekundärer Aminogruppe oder aromatische Hydroxyverbindungen mitverwendet werden.

## Claims

1. The use of diketo compounds corresponding to formula I: 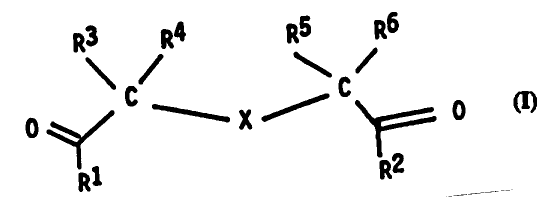 in which R¹ and R² are C₁₋₄ alkyl groups or aryl groups, R³ to R⁶ are hydrogen atoms or C₁₋₄alkyl groups or one of the substituents R³ and R⁴ is attached to one of the substituents R⁵ and R⁶, so that an isocyclic or heterocyclic saturated or unsaturated 5-, 6- or 7-membered ring is formed, and X is a methylene, ethylene or vinylene group, an N(H) group or an S or O atom, and ketals thereof for coloring kertain-containing fibers, more particularly human hair.

2. The use claimed in claim 1, characterized in that the diketo compound corresponding to formula I is selected from 2,6-diacetylpyridine and 1,3-dibenzoylpropane.

3. The use claimed in claims 1 and 2, characterized in that compounds containing a primary or secondary amino group or aromatic hydroxy compounds are also used.

## Revendications

1. Utilisation de composés dicéto de la formule I 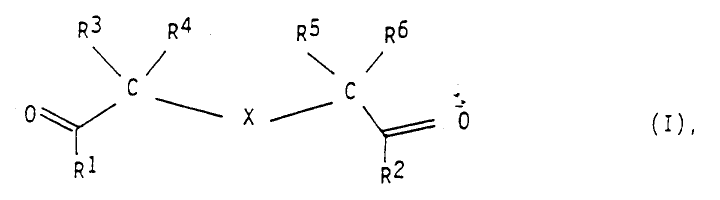 dans laquelle les radicaux R¹ et R² représentent des groupes alkyle en C₁-C₄ ou aryle, les radicaux R₃ à R₆ correspondent à l'hydrogène ou à un groupe alkyle en C₁-C₄, ou bien un des radicaux R₃ et R₄ est lié avec un des radicaux R₅ et R₆, de manière à former un cycle pentagonal, hexagonal ou heptagonal isocyclique ou hétérocyclique, saturé ou insaturé, et X est un groupe méthylène, éthyléne ou vinylène, un groupe N(H) ou un atome de S ou de O, ainsi que leurs cétals pour la coloration des fibres contenant de la kératine, en particulier les cheveux humains.

2. Utilisation selon la revendication 1, caractérisée en ce que le composé dicéto de la formule I est sélectionné parmi la 2,6-diacétylpyridine et le 1,3-dibenzoylpropane.

3. Utilisation selon la revendication 1 et 2, caractérisée en ce que des composés comportant un groupe amino primaire ou secondaire, ou des composés hydroxylés aromatiques sont co-utilisés.
